Europäisches Patentamt

European Patent Office

Office européen des brevets

(11) Publication number: **0 118 194**
**B1**

# EUROPEAN PATENT SPECIFICATION

(45) Date of publication of patent specification: 22.04.87

(51) Int. Cl.⁴: **A 61 F 2/34**

(21) Application number: **84300594.3**

(22) Date of filing: **31.01.84**

(54) Improvements in acetabular prostheses.

(30) Priority: **04.02.83 GB 8303188**

(43) Date of publication of application:
**12.09.84 Bulletin 84/37**

(45) Publication of the grant of the patent:
**22.04.87 Bulletin 87/17**

(84) Designated Contracting States:
**BE DE FR GB IT NL SE**

(56) References cited:
**EP-A-0 091 315**
**GB-A-1 296 162**
**GB-A-2 052 997**
**US-A-3 813 699**
**US-A-3 829 904**
**US-A-4 068 324**
**US-A-4 324 006**

(73) Proprietor: **Chas F Thackray Limited**
**P.O. Box 171 Park Street**
**Leeds LS2 8PB (GB)**

(72) Inventor: **Wroblewski, Boguslaw Michael**
**9 Romney Way**
**Whitley Wigan Lancashire WN1 2QQ (GB)**

(74) Representative: **Harrison, Michael Robert et al**
**URQUHART-DYKES & LORD 5th Floor Tower**
**House Merrion Way**
**Leeds LS2 8PA West Yorkshire (GB)**

Courier Press, Leamington Spa, England.

## Description

This invention relates to improvements in acetabular prostheses used in artificial hip joints.

In the surgical operation of total hip replacement, an acetabular prosthesis is inserted into an acetabulum in the human pelvis and a mating femoral component is inserted in the femur. After such a hip replacement operation one of the major problems is the danger of dislocation of the hip normally as a result of particular movements of the subject. This is particularly common after a revisionary operation.

US patent specification No. 3829904 describes an acetabular prosthesis comprising an outer surface and an inner femoral head receiving surface, which two surfaces have their spherical centres displaced and their axes of symmetry mutually angled at approximately 20°. The displacement of the centres of the two surfaces and the mutual angling of the two axes of symmetry serve to provide an increased wall thickness in the main load bearing region of the prosthesis. This is advantageous in preventing early penetration of the acetabular prosthesis by the associated femoral component, but does not offer adequate protection against dislocation, which is the aim of the present invention.

According to this invention there is provided an acetabular prosthesis including an outer surface, a femoral head receiving surface and a face which joins the edges of these two surfaces, the femoral head receiving surface comprising a blind bore extending from the face towards the outer surface, characterised in that the bore extends along an axis which is at an angle of between 47° and 36.5° to the normal to the plane defined by at least a major portion of the edge of the outer surface.

Preferably the blind bore comprises a spherical part and a cylindrical part, the radius of the cylindrical part being the same as that of the spherical part, and the centre of the spherical part being on the axis of the cylindrical part. Preferably the axis of the cylindrical part lies at an angle of 41.5° to the normal to the plane defined by the major part of the edge of the outer surface.

In many acetabular prostheses the face of the prosthesis lies on the plane defined by the edge of the outer surface, but in other protheses the face is non-planar and lies between the plane and the outer surface.

Thus the acetabular prosthesis does not limit the range of movement of the femoral component but deters dislocation since the angled spherical surface together with the face form a lip which serves to increase the distance through which the femoral head has to rotate before coming out of the blind bore.

Preferably the prosthesis is mounted in the pelvis such that the direction of the plane in which dislocation is most likely to occur is aligned with the axis of the blind bore forming the femoral head receiving surface. In this position the position where the lip is greatest is aligned with the plane in which dislocation is most likely to occur.

The angle between the axis of the cylindrical part of the femoral head receiving surface and the normal to the plane is limited by the cylindrical surface fouling the outer surface of the prosthesis when the angle is too large. The most preferable angle is 41.5°.

Preferably the face of the prosthesis is chamfered to increase the range of movement of the femoral component.

The centre of the circle surface may lie on the central axis of the prosthesis but may be offset to give an increased thickness between the femoral head receiving surface and the outer surface of the prosthesis at the point where most stress is applied.

The prosthesis may not include a flange, but preferably does include a flange.

The socket may be made of any suitable material, for instance high density polyethylene.

An example of an acetabular hip prosthesis will now be described with reference to the accompanying drawings, in which:—

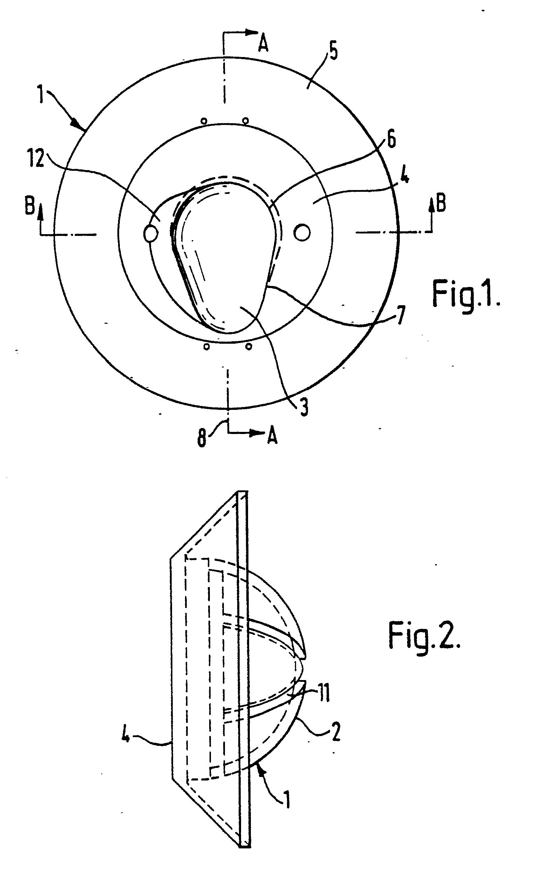

Figure 1 is a plan view of an acetabular prosthesis;

Figure 2 is a side projection of the prosthesis;

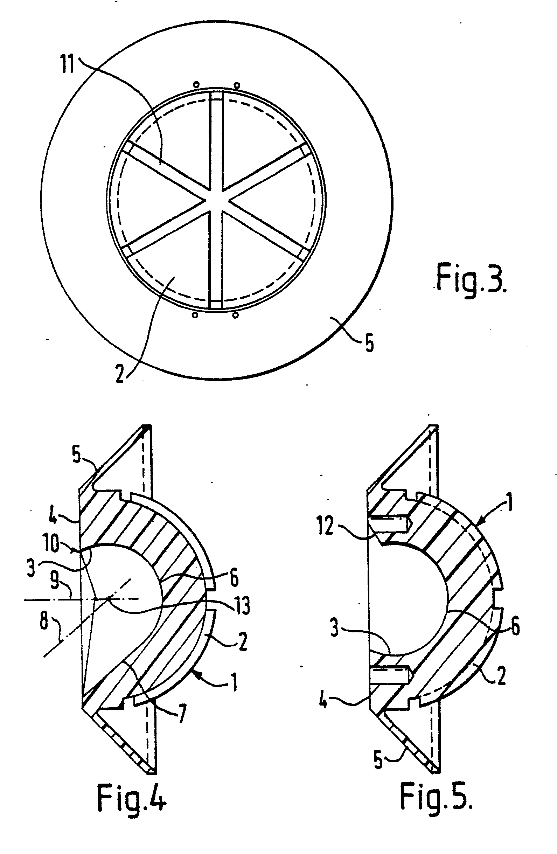

Figure 3 is a view of the outer surface of the prosthesis;

Figure 4 is a section along the line A—A of Figure 1; and

Figure 5 is a section along the line B—B of Figure 1.

A hip prosthesis 1 includes an outer surface 2, an inner femoral head receiving surface 3, a planar face 4 and an annular flange 5. The prosthesis 1 is made of high density polyethylene.

The femoral head receiving surface comprises a spherical part 6 and a cylindrical part 7. The cylindrical part 7 has the same radius as the spherical part. The centre of the spherical part 6 lies on the axis 8 of the cylindrical surface 7. The central axis 8 is at an angle to the normal 9 to the plane containing the face 4 of 41.5°. A lip 10 is formed between the edge of the spherical part 6 and the face 4 which deters dislocation of the femoral head component.

The centre 13 of the spherical part 6 of the bore lies on the central axis 9 of the prosthesis.

Grooves 11 are formed in the outer surface 2 of the prosthesis which are used to help attach the hip prosthesis to the human pelvis. A chamfer 12 is included in the face 4 to increase the range of movement of the femoral component, the chamber 12 lying substantially parallel to the axis 8 of the cylindrical surface.

The prosthesis shown is a left-handed prosthesis in which a chamfer is at the left hand side of the axis 8.

A right handed prosthesis would be of similar construction with the chamfer situated at the right hand side of the axis of the cylindrical surface.

## Claims

1. An acetabular prosthesis (1) including an outer surface (2), a femoral head receiving surface (3) and a face (4) which joins the edges of these two surfaces (2, 3), the femoral head receiving surface (3) comprising a blind bore extending from the face (4) towards the outer surface (2), characterised in that the bore extends along an axis (8) which is at an angle of between 47° and 36.5° to the normal (9) to the plane defined by at least a major portion of the edge of the outer surface (2).

2. An acetabular prosthesis according to Claim 1, further characterised in that the blind bore comprises a spherical part (6) and a cylindrical part (7), the radius of the cylindrical part (7) being the same as that of the spherical part (6), and the centre of the spherical part (6) being on the axis (8) of the cylindrical part (7).

3. An acetabular prosthesis according to Claim 2 further characterised in that the angle between the axis (8) of the cylindrical part (7) and the normal (9) to the plane is 41.5°.

4. An acetabular prosthesis according to Claim 2 or Claim 3 further characterised in that the centre (13) of the spherical part (6) is offset from the central axis of the prosthesis to give an increased thickness between the femoral head receiving surface (3) and the outer surface (2) of the prosthesis at the point where most stress is applied.

5. An acetabular prosthesis according to Claim 2 or Claim 3 further characterised in that the centre (13) of the spherical part (6) lies on the central axis of the prosthesis.

6. An acetabular prosthesis according to any one of the preceding claims, further characterised in that the face (4) joining the two surfaces (2, 3) lies on the plane defined by the edge of the outer surface (2).

7. An acetabular prosthesis according to any one of Claims 1 to 5, further characterised in that the face (4) joining the two surfaces (2, 3) is non-planar and lies between the outer surface (2) and the plane defined by the edge of the outer surface (2).

8. An acetabular prosthesis according to any one of the preceding claims further characterised in that the prosthesis is mounted in the pelvis such that the direction of the plane in which dislocation is most likely to occur is aligned with the axis (8) of the blind bore.

9. An acetabular prosthesis according to any one of the preceding claims further characterised in that the face (4) is chamfered.

10. An acetabular prosthesis according to any one of the preceding claims further characterised in that it includes a flange (5).

11. An acetabular prosthesis according to any one of the preceding claims further characterised in that it is made of high density polyethylene.

**Patentansprüche**

1. Acetabulum-Prothese (1) mit einer Außenfläche (2), einer Femoralkopf-Aufnahmefläche (3) und einer Fläche (4), die die Ränder dieser beiden Flächen (2, 3) verbindet, wobei die Femoralkopf-Aufnahmefläche (3) eine sich von der Fläche (4) zur Außenfläche (2) erstreckende Sackbohrung aufweist, dadurch gekennzeichnet, daß die Bohrung sich entlang einer Achse (8) erstreckt, die in einem Winkel zwischen 47° und 36,5° zu der Normalen (9) zu der durch mindestens einen Hauptteil des Randes der Außenfläche (2) definierten Ebene verläuft.

2. Acetabulum-Prothese nach Anspruch 1, dadurch gekennzeichnet, daß die Sackbohrung einen sphärischen Teil (6) und einen zylindrischen Teil (7) aufweist, wobei der Radius des zylindrischen Teils (7) gleich demjenigen des sphärischen Teile (6) ist und der Mittelpunkt des sphärischen Teils (6) auf der Achse (8) des zylindrischen Teils (7) liegt.

3. Acetabulum-Prothese nach Anspruch 2, dadurch gekennzeichnet, daß der Winkel zwischen der Achse (8) des zylindrischen Teils (7) und der Normalen (9) zur Ebene 41,5° beträgt.

4. Acetabulum-Prothese nach Anspruch 2 oder 3, dadurch gekennzeichnet, daß der Mittelpunkt (13) des sphärischen Teils (6) von der Mittelachse der Prothese versetzt ist, damit sich zwischen der Femoralkopf-Aufnahmefläche (3) und der Außenfläche (2) der Prothese an dem Punkt, auf den die größte Belastung wirkt, eine vergrößerte Dicke ergibt.

5. Acetabulum-Prothese nach Anspruch 2 oder 3, dadurch gekennzeichnet, daß der Mittelpunkt (13) des sphärischen Teils (6) auf der Mittelachse der Prothese liegt.

6. Acetabulum-Prothese nach einem der vorhergehenden Ansprüche, dadurch gekennzeichnet, daß die die beiden Flächen (2, 3) verbindende Fläche (4) auf der Ebene, die durch den Rand der Außenfläche (2) definiert ist, liegt.

7. Acetabulum-Prothese nach einem der Ansprüche 1 bis 5, dadurch gekennzeichnet, daß die die beiden Flächen (2, 3) verbindende Fläche (4) nicht-planar ist und zwischen der Außenfläche (2) und der durch den Rand der Außenfläche (2) definierten Ebene liegt.

8. Acetabulum-Prothese nach einem der vorhergehenden Ansprüche, dadurch gekennzeichnet, daß die Prothese derart im Becken angebracht ist, daß sich die Richtung der Ebene, in welcher eine Verschiebung am wahrscheinlichsten eintritt, in Ausrichtung mit der Achse (8) der Sackbohrung befindet.

9. Acetabulum-Prothese nach einem der vorhergehenden Ansprüche, dadurch gekennzeichnet, daß die Fläche (4) abgeschrägt ist.

10. Acetabulum-Prothese nach einem der vorhergehenden Ansprüche, dadurch gekennzeichnet, daß sie einen Flansch (5) aufweist.

11. Acetabulum-Prothese nach einem der vorhergehenden Ansprüche, dadurch gekennzeichnet, daß sie aus Polyethylen hoher Dichte besteht.

**Revendications**

1. Prothèse (1) de cotyle de l'os illiaque comprenant une surface extérieure (2), une surface (3) propre à recevoir la tête du fémur et une face (4) reliant les bords desdites surfaces (2, 3), la surface (3) étant réalisée sous la forme d'un trou borgne s'étendant à partir de la face (4) en direction de la surface (2), caractérisée en ce que l'axe géométrique dudit trou forme un angle entre 47° et 36°5 par rapport à la normale (9) à un plan défini par au moins un partie du bord de la surface extérieure (2).

2. Prothèse suivant la revendication 1, caractérisée en ce que le trou borgne comprend une partie sphérique (6) et une partie cylindrique (7), le rayon de cette partie cylindrique (7) étant le même que celui de la partie cylindrique (6), tandis que le centre de cette dernière partie se trouve sur l'axe géométrique (8) de la partie cylindrique (7).

3. Prothèse suivant la revendication 2, caractérisée en ce que l'angle formé entre l'axe géométrique (8) de la partie cylindrique (7) et la normale (9) au plan est de 41,5°.

4. Prothèse suivant l'une des revendications 2 ou 3, caractérisée en ce que le centre (13) de la partie sphérique (6) est décalé par rapport à l'axe géométrique central de la prothèse en vue d'augmenter l'épaisseur entre la surface (3) et la surface extérieure (2) de la prothèse au point où l'effort le plus important est appliqué.

5. Prothèse suivant l'une des revendications 2 ou 3, caractérisée en ce que ledit centre (13) de la partie sphérique se trouve sur l'axe géométrique de la prothèse.

6. Prothèse suivant l'une quelconque des revendications précédentes caractérisée en ce que la face (4) reliant les deux surfaces (2, 3) se trouve dans le plan défini par le rebord de la surface extérieure (2).

7. Prothèse suivant l'une quelconque des revendications 1 à 5, caractérisée en ce que la face (4) reliant les deux surfaces (2, 3) n'est pas plane et se trouve entre la surface extérieure (2) et le plan défini par le bord de la surface extérieure (2).

8. Prothèse suivant l'une quelconque des revendications précédentes, caractérisée en ce que la prothèse est placée dans le bassin afin que la direction du plan dans lequel il est le plus vraisemblable que la dislocation s'effectue, soit alignée avec l'axe géométrique (8) du trou borgne.

9. Prothèse suivant l'une quelconque des revendications précédentes, caractérisée en ce que la face (4) est chanfreinée.

10. Prothèse suivant l'une quelconque des revendications précédentes, caractérisée en ce qu'elle comporte une bride (5).

11. Prothèse suivant l'une quelconque des revendications précédentes, caractérisée en ce qu'elle est réalisée en polyéthylène haute densité.

0 118 194

Fig.1.

Fig.2.

11

Fig.3.

5

2

5

4

10

3

9

8

6

13

2

1

7

Fig.4

12

1

3

6

2

4

5

Fig.5.

2